# EUROPEAN PATENT APPLICATION

(11) **EP 1 598 359 A1**
(43) Date of publication of application: **23.11.2005**
(21) Application number: 05090144.6
(22) Date of filing: 19.05.2005
(51) Int. Cl.: C07D 498/22, C07D 241/04, C06B 25/34

(54) **Process for synthesis of dinitrotetraoxadiazisowursitane (DTIW)**

(30) Priority: 19.05.2004 IL 16207104
(71) Applicant: RAFAEL - ARMAMENT DEVELOPMENT AUTHORITY LTD., Haifa 31021 (IL)
(72) Inventor: Gottlieb, Levi, Haifa 34384 (IL); Korogodsky, Gad, Kiryat Bialik 27204 (IL); Evron, Yaval, 15295 (IL)
(74) Representative: Hannig, Wolf-Dieter

(57) **Abstract**

A process for producing dinitrotetraoxadiazaisowurzitane (DTIW), including the steps of: (a) introducing a solid material including 1,4-diformyl-2,3,5,6-tetrahydroxypiperazine (THDFP) and a nitrating acid to a reaction vessel, and (b) reacting the THDFP with the nitrating acid in a reaction stage so as to form a solid product containing DTIW, wherein the nitrating acid includes nitric acid and sulfuric acid, and wherein a weight fraction of the nitric acid in the nitrating acid is within a range of 0.40 to 0.55, a weight fraction of the sulfuric acid is within a range of 0.60 to 0.45, and a weight fraction of water in the nitrating acid, with respect to the nitric and sulfuric acids, is less than 0.015.

## Description

### FIELD AND BACKGROUND OF THE INVENTION

The present invention relates to a method of producing explosives and more specifically, to an improved method of producing dinitrotetraoxadiazaisowurzitane, DTIW and a precursor thereof, 1,4-diformyl-2,3,5,6-tetrahydroxypiperazine (THDFP).

DTIW, also known as "TEX", is a low sensitivity, high energy explosive that has been widely suggested for use in insensitive high explosive compositions complying with the insensitive munitions requirements. The structure of DTIW and the structure of the THDFP precursor are provided below:

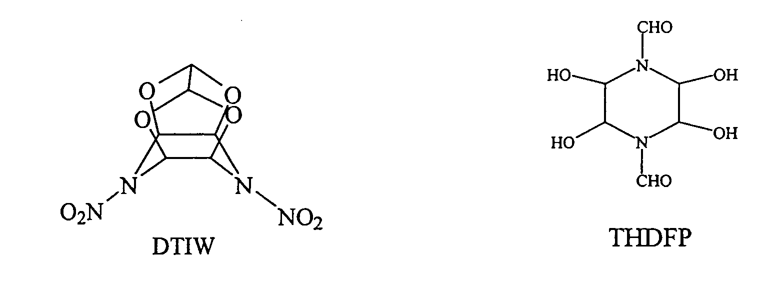

THDFP is produced in the base-catalyzed condensation of aqueous glyoxal and formamide. A typical condensation is carried out by dissolving formamide in 40% w/w aqueous glyoxal in a molar ratio of 1:1 or 1:2, cooling to 0°C, and adding a base (NaHCO₃ or NaOH), to achieve a pH of 8-10. The synthesis of THDFP is often characterized by extremely long reaction times of 25-72 hours. The yields in this reaction step of these syntheses vary widely, from 28% to 81%.

Since THDFP is insoluble in most organic solvents, purification of the material by standard techniques is practically impossible, and attempts to purify the product in hot polar solvents result in decomposition of a substantial amount of the material. The thermal behavior is the main indication of purity. Heating the solid material has been reported to cause blackening of the solid, followed by decomposition thereof, according to inconsistent data in the literature. Vail, et al. (*J. Org. Chem*. 1965, 30, pp. 1195-1199), discloses that THDFP decomposes at about 225 °C, but this extremely high decomposition temperature has not been reproduced in subsequent work. Indeed, it is reported in subsequent work that THDFP darkens above 190°C without further change or decomposes between 190°C and 210°C.

Talwar, et al., in "TEX: The New Insensitive High Explosive" (*Defense Science Journal*, 2002, Vol. 52), teaches a THDFP production process in which formamide is added dropwise to glyoxal in a 1:1.7 molar ratio, over 20 minutes. Sodium bicarbonate (NaHCO₃) is then added to adjust the pH of the mixture to 8, and the reaction mixture is stirred for 6 hours. Diformamidoethanediol (DFED) begins to precipitate out of solution after the first 15 minutes of the 6-hour period. The reaction mixture is then allowed to stand for 25 hours at room temperature, with occasional stirring. After filtration, the product cake is washed with cold water and methanol. A milky white THDFP product is obtained, the reported yield being 81%. The method requires a heavy excess of formamide, and the reaction time of 25 hours is prohibitively high. It must be emphasized that the chemical purity achieved by Talwar, et al., appears to be poor, as evidenced by the low decomposition temperature.

Vedachalam, et al. (*J. Chem. Soc.* 1991, 56, 3413-3419), discloses a method of producing THDFP in which formamide is added to glyoxal at a stoichiometric, equimolar ratio. After adding a NaOH solution to adjust the pH of the mixture to 8.5, the temperature of the mixture was allowed to rise to 30°C over the course of 30 minutes. The mixture was then stirred for 4 hours and allowed to stand for 3 days. A first crop of THDFP was collected by filtration. The pH of the remaining mixture was adjusted to 9, and subsequently, the mixture was controlled at 25°C for 5 hours by cooling in ice water, to obtain a second crop of THDFP. The crude THDFP product was extensively washed with water, dried, and purified by twice digesting the crude product with hot aqueous dimethylformamide, washing and drying to obtain a THDFP product. The yield was 66%. The method taught by Vedachalam, et al., is lengthy, requires extensive purification procedures, and achieves an unsatisfactory yield.

Vail, et al. (*J. Org. Chem*. 1965, 30, 1195-1199), discloses a general procedure of adding monoamides to glyoxal at various molar ratios to produce N,N'-dihydroxyethylenebisamides. In a particular series of experiments, formamide was dissolved in a solution of glyoxal, the molar ratio of formamide to glyoxal being 1:2. Individual experiments were conducted at a pH as low as 4.5 and as high as 10, and within a temperature range of 0°C to 25°C. The reaction mixture was allowed to stand for 1-2 hours. Yields of up to 60% were obtained. Vail, et al., fails to disclose optimal pH and temperature ranges for the synthesis of THDFP, nor is a satisfactory yield achieved.

Currie, et al. (J. Chem. Soc. 1967, p. 491), discloses a method of producing THDFP in which formamide and glyoxal in a ratio of 2 moles of formamide to 1 mole of glyoxal are mixed. Sodium bicarbonate is then added to bring the pH to 8, and stirring is continued until only a trace of solid remains. The mixture is cooled to maintain the temperature below 25°C during the mixing and subsequent reaction period. After standing at room temperature for 3 days, with occasional stirring, most of the reaction mixture crystallizes out. Methanol is introduced to facilitate the filtration, and the solids are filtered off, washed with methanol, and dried to obtain the crude THDFP product. The solids are further purified by adding boiling 0.1 N HCl, stirring at the boiling point for 5 minutes, cooling rapidly, and maintaining the mixture at 0°C for 2 hours. The yield of THDFP obtained using this method was 27.4%. The process appears to produce a relatively pure product, however, the procedure is arduous, requires rapid cooling to low temperatures and, results in an exceptionally low yield.

In the above-described prior art methods, adding a large excess of formamide results in characteristic THDFP yields of about 25-80%, and also produces an unreasonably large fraction of a by-product, 1,2-diformylamino-1,2-dihydroxyethane. Basic condensations at a 1:1 ratio between the reactants may improve the yield, however, the reproducibility and purity of the material prepared by any of the literature methods have been found to be unsatisfactory.

The synthesis of DTIW via THDPF was first reported by Ramakrishnan et al. (Heterocycles, 1990, 31, 479-480). The synthesis is based on the logical assumption that THDPF should condense in acidic conditions with a tetrahydroxyethane derivative (i.e., a glyoxal equivalent) to form the tetraoxadiamido cage structure. This step is succeeded by a nitration step that yields DTIW, as follows: 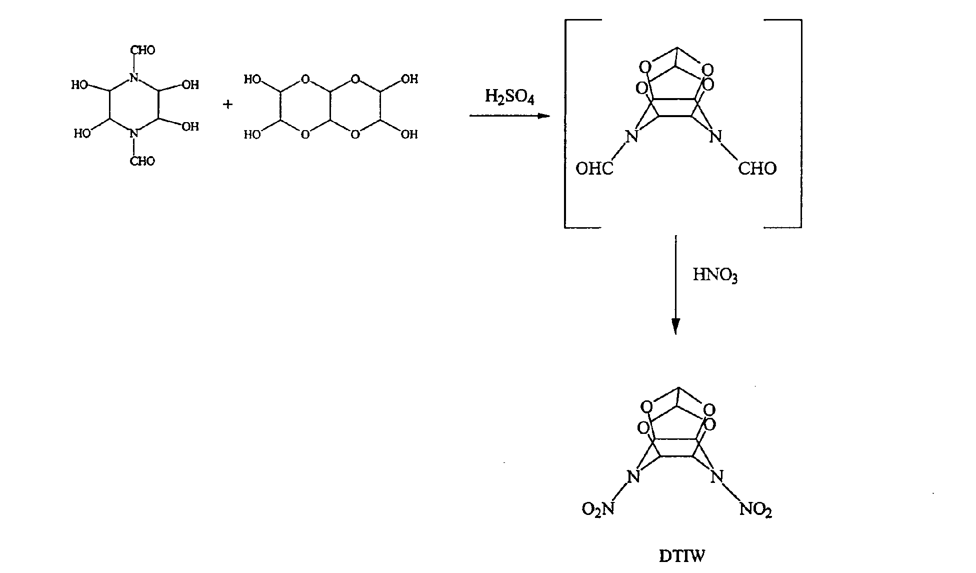 The novelty of the Ramakrishnan process notwithstanding, the product obtained is highly impure, as indicated by low decomposition temperature (>250°C) of the material. Reported attempts to repeat the procedure of Ramakrishnan confirm that the obtained DTIW is extremely impure.

A modified synthesis route for converting THDFP to DTIW is disclosed in U.S. Pat. No. 5,498,711, the complete disclosure of which is incorporated herein by reference. DTIW is synthesized by reacting 1.4-diformyl-2,3,5,6-tetrahydroxypiperazine and derivatives thereof with a strong acid and a nitrate source at temperatures greater than ambient temperature (e.g., 50°C to 70°C). The preferred strong acid and preferred nitrate source are sulfuric acid and nitric acid, respectively. The reaction is exothermic and is allowed to continue for two to three hours. The mixture is then poured onto ice, and a solid precipitate is isolated and washed to give a mixture containing the DTIW.

Purification can then be accomplished by heating the reaction product in nitric acid, washing with methanol, and/or washing with a base to neutralize excess acid. The pure product may be obtained by recrystallization.

The synthesis route reported in the '711 patent produces DTIW in yields and purities that constitute improvements over the previously-known art. However, as is evident from the use of NOₓ scavengers, the method of the '711 patent results in the generation of NOₓ by-product gases. The generation of NOₓ gases, such as NO₂, is an autocatalytic reaction that becomes rapid within a relatively brief period of time, causing fume-off of reactants and product, and lowering thereby, the product yield. Also, there is a tangible safety risk associated with an uncontrolled, exothermic reaction due to autocatalytic generation and release of NO₂. These deficiencies become even more acute when the production is scaled-up, such that the process appears to be highly unsuitable for implementing as an industrial process.

International Patent WO 00/09509 to Hajik, et al., reports that the process taught by the '711 patent, in which the reaction temperature is controlled at a relatively low temperature (preferably below 50°C), results in excessive NOₓ generation, fume off, and low yields of the DTIW product.

Hajik, et al., attempts to ameliorate some of the problems inherent in the process taught by the '711 patent, by adding a mixture of THDFP and the urea scavenger at a relatively-high temperature of 55-60°C. However, the reaction is violent, thereby necessitating a lengthy and careful addition if the reaction is to be scaled-up to an industrial scale. It is disclosed that a 7-hour period was required to add 618g of THDFP, in portions of 30g, to the reaction mixture. The process achieves a characteristic yield of approximately 27% (w/w based on THDFP), which leaves room for improvement.

The above-described processes display extreme sensitivity to the presence of water, which reacts within the reaction mixture to release NO₂. This autocatalytic reaction further decreases the nitrating power of the mixture, and ultimately reduces the yield.

The above-mentioned deficiencies have also been observed by recently-issued U.S. Pat. No. 6,512,113 to Sanderson, et al. U.S. Pat. No. 6,512,113 goes on to disclose a synthesis that, unlike conventional processes, is conducted in a medium that is free or substantially free of a strong acid other than nitric acid. U.S. Pat. No. 6,512,113 teaches that "counter-intuitively, it has been found that the elimination of a strong acid, such as sulfuric acid, increases the TEX formation rate." It was surprisingly discovered, that high concentrations of strong acids, such as sulfuric acid, promote foaming during exothermic stages of the reaction.

The process disclosed by U.S. Pat. No. 6,512,113 is not, however, free of the foaming/fume-off phenomena that characterized the previously-known processes. In one of the Examples provided by U.S. Pat. No. 6,512,113, it is reported that the temperature was allowed to rise to 81°C. However, even though the temperature was brought down after the initial exotherm, a fume-off occurred that could not be quenched by cooling and the reactor could not be rapidly drained into a cold tank due to the gas evolution in the boiling acid.

The control of the synthesis procedure taught by U.S. Pat. No. 6,512,113 is extremely delicate. After a temperature rise of only 1°C is observed, the reaction solution is quickly cooled to -5°C to 0°C using ice water or an ice/acetone mixture. Moreover, since foaming/fume-off phenomena tend to increase appreciably in large-scale industrial production, such problems in small-scale laboratory production strongly indicate that the process is unsuitable for commercial implementation.

There is therefore a recognized need for, and it would be highly advantageous to have, a method for the production of DTIW via THDFP that is safe and robust, achieves a higher yield, and is more conducive to scale-up than methods known heretofore. It would be of further advantage if such a method would also be simple and require characteristically-short reaction times.

### SUMMARY OF THE INVENTION

The present invention is an improved method of synthesizing dinitrotetraoxadiazaisowurzitane, DTIW, via a precursor, 1,4-diformyl-2,3,5,6-tetrahydroxypiperazine (THDFP).

According to the teachings of the present invention there is provided a process for producing DTIW, including the steps of: (a) combining glyoxal and a base to produce a mixture; (b) subsequently to step (a), adding formamide to the mixture, and (c) reacting the formamide with the mixture in a reaction stage to produce a solid product containing THDFP.

According to yet another aspect of the present invention there is provided a process for producing DTIW, including the steps of: (a) providing reactants including glyoxal, formamide, and a base, and (b) reacting the reactants in a reaction stage to produce a solid product containing THDFP, wherein the reaction stage is controlled such that the reacting is conducted at a pH above 10.5.

According to still further features in the described preferred embodiments, the reaction stage is controlled such that the reacting is conducted at a pH above 11, preferably at a pH between 11 and 13.

According to still further features in the described preferred embodiments, the reaction stage is controlled such that the reacting is conducted at a temperature above 30°C, preferably within a temperature range between 35°C and 50°C.

According to still further features in the described preferred embodiments, the process further includes the step of: (d) reacting the solid product with a nitrating acid to produce the DTIW.

According to still further features in the described preferred embodiments, the solid product is directly added to the nitrating acid.

According to still further features in the described preferred embodiments, the nitrating acid includes nitric acid and sulfuric acid.

According to yet another aspect of the present invention there is provided a process for producing DTIW, including the steps of: (a) introducing a solid material including THDFP and a nitrating acid to a reaction vessel, and (b) reacting the THDFP with the nitrating acid in a reaction stage so as to form a solid product containing DTIW, wherein the nitrating acid includes nitric acid and sulfuric acid, and wherein a weight fraction of the nitric acid in the nitrating acid is within a range of 0.40 to 0.55, a weight fraction of the sulfuric acid is within a range of 0.60 to 0.45, and a weight fraction of water in the nitrating acid, with respect to the nitric acid and the sulfuric acid, is less than 0.015.

Preferably, the weight fraction of water is less than 0.012, more preferably, less than 0.010, and most preferably, less than 0.005.

According to still further features in the described preferred embodiments, a weight to weight ratio of the nitrating acid to the THDFP is greater than 8.5 to 1, preferably greater than 10 to 1, and most preferably between 12 to 1 and 15 to 1.

According to still further features in the described preferred embodiments, the reaction to produce DTIW is conducted at a temperature above 40°C, preferably between 40°C to 65°C, and most preferably between 45°C to 55°C.

The present invention successfully addresses the shortcomings of the existing technologies by providing a process for the simple, reliable, and inexpensive large-scale production of DTIW.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a block diagram illustrating a method of DTIW production according to the present invention, and
FIG. 2 is a graph illustrating the relationship of temperature vs. time for the inventive DTIW reaction stage, over the course of several runs, in which a non-violent thermal behavior is achieved.

### DESCRIPTION OF THE PREFERRED EMBODIMENTS

The present invention is an improved method of synthesizing dinitrotetraoxadiazaisowurzitane, DTIW, via a precursor, 1,4-diformyl-2,3,5,6-tetrahydroxypiperazine (THDFP).

The principles and operation of this process according to the present invention may be better understood with reference to the drawings and the accompanying description.

Before explaining at least one embodiment of the invention in detail, it is to be understood that the invention is not limited in its application to the details of construction and the arrangement of the components set forth in the following description or illustrated in the drawing. The invention is capable of other embodiments or of being practiced or carried out in various ways. Also, it is to be understood that the phraseology and terminology employed herein is for the purpose of description and should not be regarded as limiting.

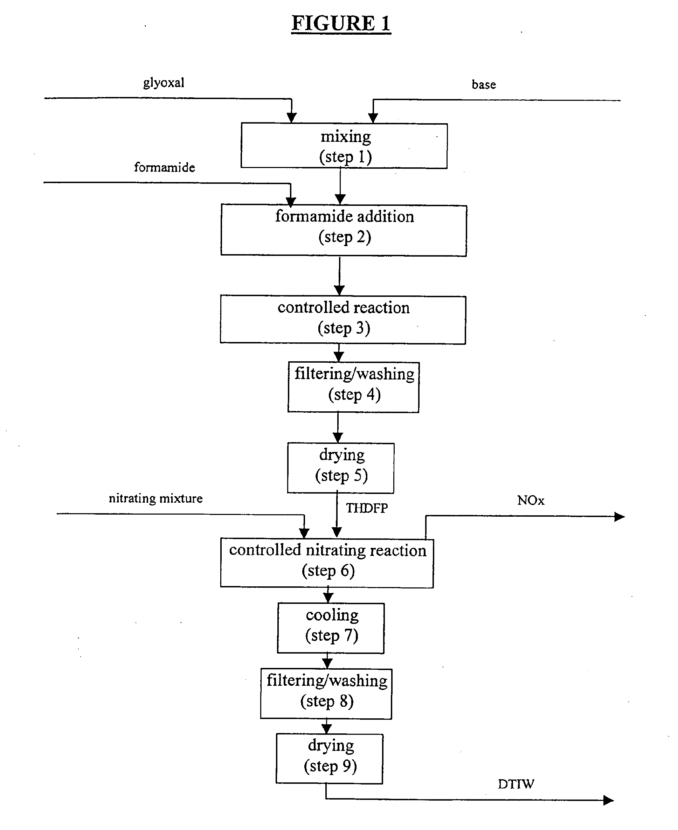

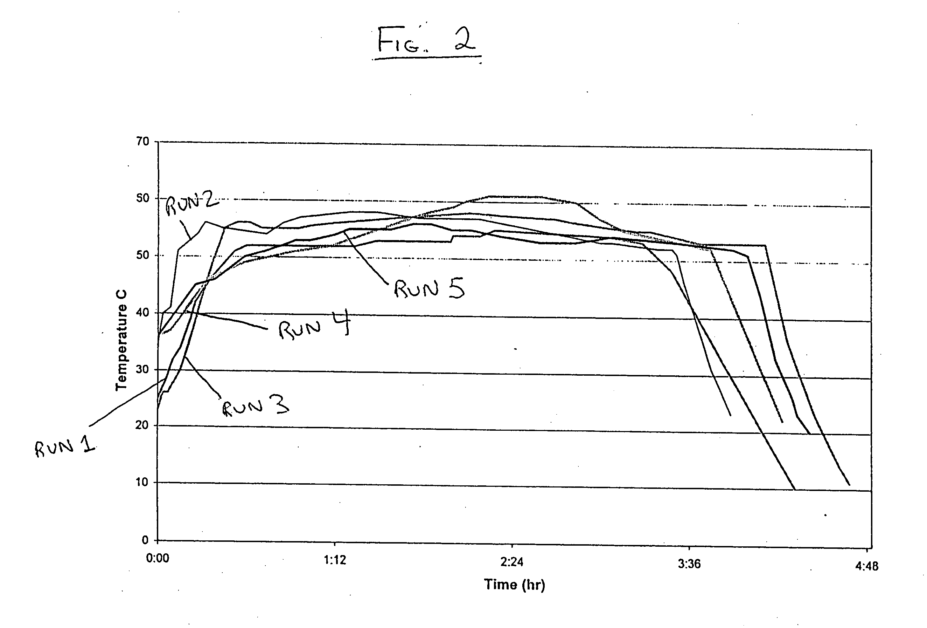

Referring now to the drawings, Figure 1 is a block diagram illustrating one embodiment of the method of the present invention. In step 1, glyoxal is combined with a base, in an aqueous medium, so as to raise the pH of the mixture above 10, and preferably, to a pH within the range of 10-13. More preferably, the pH of the mixture should exceed 10.5, and most preferably, the pH should be in the range of 11-13.

In step **2,** formamide is introduced to the basic, glyoxal-containing medium formed in step 1, and the reaction of formamide with the glyoxal to produce THDFP ensues. In step 3, the reaction mixture is controlled at a temperature preferably between 25°C and 50°C, and, more preferably, between 30°C and 45°C. The reaction is allowed to proceed within the controlled temperature range, for approximately 30 minutes to 4 hours, and, more preferably, 40 minutes to 1 hour. The reaction is then terminated by filtering the white solid that has precipitated in the reaction. Optionally, the solid is washed with water and/or with acetone (step **4**) prior to drying (step **5**).

Preferably, the formamide and glyoxal reactants are added in an equimolar, or near-equimolar ratio.

The base may be aqueous NaOH, for example, having a concentration of 10M.

The introduction of formamide should be performed slowly, to avoid reaching high temperatures (above ~50°C), at which THDFP tends to decompose in basic, aqueous media. Alternatively, the glyoxal-containing medium formed in step **1** can be maintained at a low temperature (e.g., 5°-20°C), such that heat produced by the exothermic formation of THDFP supplies the sensible heat for raising the temperature of the reaction mixture to a pre-determined value (e.g., 35°C - 45°C). A cooling system is used, as necessary, to maintain the temperature of the reaction mixture below another pre-determined value (e.g., 45°C - 50°C).

Thus, as compared with the various methods of the prior art, the methods of the instant invention allow for an extremely short residence time with respect to most methods of the prior art. The inventive method achieves a yield of 80-85%, typically within one hour. The THDFP produced is sufficiently pure for the subsequent DTIW production, without dissolution and recrytallization. Moreover, the results achieved have been found to be reproducible, and the unit operations are eminently suitable for scaling-up to an industrial process.

The manifest improvement in the product quality and the marked decrease in reaction time are attributed to the following:
1) adding the base to the glyoxal prior to introducing the formamide, such that the formamide contacts a homogeneous, basic solution;
2) maintaining the pH of the reaction mixture above 10, and most preferably, between 11 and 13, which accelerates the rate of THDFP production, and enhances selectivity, and
3) controlling the temperature between 25°C and 50°C, and, more preferably, between 30°C and 45°C, to accelerates the rate of THDFP production, but without causing undue decomposition of the THDFP.

The improved product quality can allow the THDFP produced to be directly added to the nitrating reaction stage (DTIW production) without having to undergo complex, expensive purification processes such as dissolution and recrystallization, as in various known processes.

In the reaction of THDFP in a nitrating medium to produce DTIW (step 6), the method of the present invention eliminates or considerably diminishes the possibility of autocatalytic production of NO₂ and fume-off. As a result, process safety is improved, the yield and purity of the explosive material are enhanced, and reasonable reaction times are achieved.

Many experimental factors have a profound influence on the reaction behavior: the ratio of sulfuric acid to nitric acid, concentration of nitric acid, or the presence of water, amounts of acids relative to THDFP, the mode of addition, addition temperature, and reaction temperature.

We have discovered that the most important criterion influencing the safety aspect of the DTIW-producing reaction is the ratio of nitric acid/sulfuric acid/water. In sharp contrast to the teachings of recently-issued U.S. Pat. No. 6,512,113 to Sanderson, et al., a high ratio of sulfuric acid to nitric acid (in the mixed acid) is actually advantageous, provided that the water concentration in the nitrating mixture is sufficiently low. Such conditions enable the reaction to proceed in a safe, easily-controllable, robust, and reproducible manner, without the need for scavengers, such that the reaction is highly conducive to scale-up.

Preferably, the weight fraction of nitric acid is within the range of 0.40 to 0.55, the weight fraction of sulfuric acid is within the range of 0.60 to 0.45, and the weight fraction of water with respect to the nitric acid and the sulfuric acid is less than 0.015. More preferably, the weight fraction of nitric acid is within the range of 0.40 to 0.52 (the corresponding weight fraction of sulfuric acid being within the range of 0.60 to 0.48), and most preferably, the weight fraction of nitric acid is within the range of 0.42 to 0.50 (the corresponding weight fraction of sulfuric acid being within the range of 0.58 to 0.50).

With regard to the water content of the mixed acid, the weight fraction of water with respect to the nitric acid and sulfuric acid is preferably less than 0.012, more preferably less than 0.010, and most preferably, less than 0.005.

In addition, the ratio of mixed acid to the THDFP reactant is preferably increased with respect to the ratios disclosed by presently-known methods. The increased ratio of mixed acid to THDFP reactant acts, inter alia, as a considerable heat sink capable of dissipating the heat of the reaction. The increased amount of sulfuric acid in the mixed acid also reduces the solubility of DTIW in the reaction mixture, which results in a moderate increase in the yield.

It should be emphasized that in the process of the present invention, the nitrating solution can be prepared from inexpensive, commercial 100% nitric acid and concentrated sulfuric acid using standard methods, obviating the need for purification or distillation of the acid, or for the addition of water (as in known processes).

The entire amount of THDPF is added to the nitrating mixture in a controlled nitrating reaction (step 6), preferably at ambient temperatures. Only a slight exothermic reaction is observed. The preferred range of ratios of mixed acid to THDFP is about 8.5:1 to about 20:1, more preferably 10:1 to 16:1, and most preferably 12:1 to 15:1, on a weight-to-weight basis. The reaction mixture is then heated to a temperature above 40°C, preferably to 45-65°C, and most preferably to 45-55°C. The reaction mixture is maintained at this temperature for approximately 3 hours. In the reaction, which is slightly exothermic, a negligible gas release is observed, having a faint red-brown coloration typical of NO₂.

The solid THDFP completely disappears and DTIW crystallizes from the reaction mixture. When gas release is no longer observed, the heating is terminated and the reaction is slowly cooled (step 7) to the ambient temperature. The solid DTIW is filtered (step 8) from the reaction mixture, and washed (step 8) to neutral pH with water and base. Subsequently, the DTIW product is washed with methanol and dried in air (step 9).

The material thus obtained is 97-98% pure, as confirmed by HPLC analysis, and has an average particle size of about 40-50µm. The reaction in these conditions is very stable and has been conducted both on a small scale (based on 1-2 liters of the nitrating mixture) and on a larger scale (8-10 liters of the nitrating mixture). The reaction proceeds in a safe and extremely consistent fashion, yielding results: yield, purity, and particle size, which are also consistent and favorable.

In Figure 2, the temperature profiles of five runs are graphically provided. The reactions, which were conducted in a 16-liter Pfaudler® reactor (using 10 liters of the mixed acid), manifestly exhibited a non-violent thermal behavior.

Although there is room for optimizing the inventive process, the process has yielded DTIW in 30% w/w concentration based on THDFP, or in 36% concentration based on assumption that each mole of THDFP should give 2/3 moles of DTIW. The yield obtained is an improvement over yields obtained in known processes, and is obtained in a reaction procedure that is mild, safe and reproducible, and releases insignificant amounts of NO₂ gas.

### EXAMPLES

Reference is now made to the following examples, which together with the above description, illustrate the invention in a non-limiting fashion.

### Example 1- THDFP Production

300ml of an aqueous 20% w/w NaOH solution were slowly added to 3 kilograms of an aqueous glyoxal solution (40% w/w; 20.7 mole) in a 12-liter flask equipped with a mechanical stirrer. After cooling the reaction mixture to 10°C, 930g of formamide (20.7 mole) were rapidly added dropwise within a period of 20-30 minutes. Subsequent to the formamide addition, the cooling was suspended, and the temperature was allowed to rise. When the reaction temperature reached 37°C, the cooling was reapplied so as to maintain the temperature below 45°C. After an hour, the reaction was terminated by filtering the white solid that had precipitated in the flask. The solid was washed twice with water and then with acetone, and was dried overnight. 1.8 kilogram of dry THDFP product was produced, corresponding to a yield of 83%. The product had a melting (and decomposition) point of 205°C. The product material was found to contain 34.72% C, 4.90% H, and 13.42% N, a composition that closely approaches the theoretical, calculated elemental analysis for C₆H₁₀N₂O₆ (34.96% C, 4.89% H, 13.59% N).

### Example 2: DTIW Production from THDFP

1 liter of concentrated sulfuric acid was slowly added to 1 liter of concentrated, 100% nitric acid (non-distilled), stirred in a 3-liter jacketed flask cooled by circulating tap water. 250 grams of THDFP were then added, in a single portion, to the acid mixture at ambient temperature. The reaction mixture was then heated to 48-50°C. A very weak exothermic reaction ensued, raising the temperature 2°C-4°C above the average temperature of the applied heating. A faint, red-brown fuming, characteristic of NO₂ evolution, was observed. After the fuming ceased, the heating was terminated, and the mixture was slowly cooled to 15-25°C. The product was filtered from the reaction mixture, washed several times with water, and was then washed in series with a saturated NaHCO₃ solution, water, and methanol, respectively. The filter cake, after being dried overnight, weighed 76.5 grams. The product containing more than 99% DTIW, such that a DTIW yield of 30.6% based on the THDFP weight (or a DTIW yield of 36.1% based on the assumed stoichiometry) was attained.

The reaction was reproduced twice, using the same quantities of the starting materials, and yielding 77.5g and 77.0g DTIW, respectively.

### Example 3- DTIW Production on a Larger Scale

5 liters of concentrated sulfuric acid was slowly added dropwise to 5 liters of concentrated, 100% nitric acid (non-distilled), stirred in a 16-liter Pfaudler®-type reactor cooled by circulating tap water. 1,250 grams of THDFP were then added, in substantially a single portion, to the acid mixture at ambient temperature. The reaction mixture was then heated to 48-50°C. A very weak exothermic reaction ensued, raising the temperature 3°C-8°C above the average temperature of the applied heating. A faint, red-brown fuming was observed, as is characteristic of NO₂ evolution. The fumes were passed through an empty trap and subsequently through a trap filled with saturated NaHCO₃ solution before being vented to the hood exhaust.

When the fuming ceased, the heating was terminated, and the mixture was slowly cooled to 18°C. The product was filtered from the reaction mixture, washed several times with water, and was then washed in series with a saturated NaHCO₃ solution, water, and methanol, respectively. The filter cake, after being dried overnight, weighed 380.0 grams. The product containing 97- 98% DTIW, such that a DTIW yield of 30.4% based on the THDFP weight (or a DTIW yield of 36.0% based on the assumed stoichiometry) was attained.

The reaction was reproduced several times, using the same quantities of the starting materials, and yielding 380-385g DTIW.

As used herein in the specification and in the claims section that follows, the term "directly" and the like, used in conjunction with the formation of THDFP, refers to the formation of THDFP from glyoxal, without prior formation of any intermediate species.

As used herein in the specification and in the claims section that follows, the term "directly adding" and the like, used in conjunction with the introduction of THDFP to the controlled nitrating reaction, refers to THDFP that is added to the nitrating reaction without undergoing dissolution, recrystallization, and similar purification steps.

As used herein in the specification and in the claims section that follows, the term "directly filtering" and the like, refers to the separation of DTIW from the mother liquor thereof without an intermediate quenching step.

As used herein in the specification and in the claims section that follows, the term "weight fraction of nitric acid", and the like, with respect to a nitrating acid mixture, refers to the weight of pure (100%) nitric acid in relation to the total weight of the nitric acid and the pure sulfuric acid, the weight of both acids being calculated on a 100% pure acid basis.

Similarly, the term "weight fraction of sulfuric acid", and the like, with respect to a nitrating acid mixture, refers to the weight of pure (100%) sulfuric acid in relation to the total weight of the nitric acid and the pure sulfuric acid, the weight of both acids being calculated on a 100% pure acid basis.

As used herein in the specification and in the claims section that follows, the term "weight fraction of water", and the like, with respect to a nitrating acid mixture, refers to the weight of water in the nitrating acid with respect to the total weight of nitric acid and sulfuric acid, the weight of both acids being calculated on a 100% pure acid basis.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A process for producing dinitrotetraoxadiazaisowurzitane (DTIW), comprising the steps of:
(a) combining glyoxal and a base to produce a mixture;
(b) subsequently to step (a), adding formamide to said mixture, and
(c) reacting said formamide with said mixture in a reaction stage to produce a solid product containing 1,4-diformyl-2,3,5,6-tetrahydroxypiperazine.

2. The process of claim 1, wherein said reaction stage is controlled such that said reacting is conducted at a pH above 10.5.

3. The process of claim 1, wherein said reaction stage is controlled such that said reacting is conducted at a pH above 11.

4. The process of claim 1, wherein said reaction stage is controlled such that said reacting is conducted at a temperature above 30°C.

5. The process of claim 1, wherein said reaction stage is controlled such that said reacting is conducted at a temperature range between 35°C and 50°C.

6. The process of claim 1, further comprising the step of:
(d) reacting said solid product with a nitrating acid to produce the DTIW.

7. The process of claim 6, wherein said solid product is directly added to said nitrating acid.

8. The process of claim 6, wherein said nitrating acid includes nitric acid and sulfuric acid.

9. The process of claim 6, wherein a weight-to-weight ratio of said nitrating acid to said THDFP is greater than 8.5 to 1.

10. The process of claim 9, wherein said ratio is greater than 10 to 1.

11. The process of claim 9, wherein said ratio is greater than 12 to 1 and less than 15 to 1.

12. The process of claim 6, wherein said reacting with said nitrating acid is conducted at a temperature above 40°C.

13. The process of claim 6, wherein said reacting with said nitrating acid is conducted at a temperature above 45°C.

14. A process for producing dinitrotetraoxadiazaisowurzitane (DTIW), comprising the steps of:
(a) providing reactants including glyoxal, formamide, and a base, and
(b) reacting said reactants in a reaction stage to produce a solid product containing 1,4-diformyl-2,3,5,6-tetrahydroxypiperazine,
wherein said reaction stage is controlled such that said reacting is conducted at a pH above 10.5.

15. The process of claim 14, wherein said reaction stage is controlled such that said reacting is conducted at a pH above 11.

16. The process of claim 14, wherein said reaction stage is controlled such that said reacting is conducted at a temperature above 30°C.

17. The process of claim 14, wherein said reaction stage is controlled such that said reacting is conducted at a temperature range between 35°C and 50°C.

18. The process of claim 14, further comprising the step of:
(c) reacting said solid product with a nitrating acid to produce the DTIW.

19. The process of claim 18, wherein said solid product is directly added to said nitrating acid.

20. The process of claim 18, wherein said nitrating acid contains nitric acid and sulfuric acid.

21. The process of claim 18, wherein a weight-to-weight ratio of said nitrating acid to said THDFP is greater than 8.5 to 1.

22. The process of claim 21, wherein said ratio is greater than 10 to 1.

23. The process of claim 21, wherein said ratio is greater than 12 to 1 and less than 15 to 1.

24. The process of claim 18, wherein said reacting is conducted at a temperature above 40°C.

25. The process of claim 18, wherein said reacting is conducted at a temperature above 45°C.

26. The process of claim 18, wherein said reacting is conducted at a temperature within the range of 40°C to 65°C.

27. The process of claim 18, wherein said reacting is conducted at a temperature within the range of 45°C to 55°C.

28. The process of claim 18, wherein said nitrating acid includes nitric acid and sulfuric acid,
and wherein a weight fraction of said nitric acid in said nitrating acid is within a range of 0.35 to 0.55, a weight fraction of said sulfuric acid is within a range of 0.65 to 0.45, and a weight fraction of water in said nitrating acid, with respect to said nitric acid and said sulfuric acid, is less than 0.015.

29. The process of claim 28, wherein said weight fraction of water is less than 0.010.

30. The process of claim 28, wherein said weight fraction of water is less than 0.005.

31. A process for producing dinitrotetraoxadiazaisowurzitane (DTIW), comprising the steps of:
(a) introducing a solid material including 1,4-diformyl-2,3,5,6-tetrahydroxypiperazine (THDFP) and a nitrating acid to a reaction vessel, and
(b) reacting said THDFP with said nitrating acid in a reaction stage so as to form a solid product containing DTIW,
wherein said nitrating acid includes nitric acid and sulfuric acid,
and wherein a weight fraction of said nitric acid in said nitrating acid is within a range of 0.40 to 0.55, a weight fraction of said sulfuric acid is within a range of 0.60 to 0.45, and a weight fraction of water in said nitrating acid, with respect to said nitric acid and said sulfuric acid, is less than 0.015.

32. The process of claim 31, wherein said weight fraction of water is less than 0.012.

33. The process of claim 31, wherein said weight fraction of water is less than 0.010.

34. The process of claim 31, wherein said weight fraction of water is less than 0.005.

35. The process of claim 31, wherein a weight to weight ratio of said nitrating acid to said THDFP is greater than 8.5 to 1.

36. The process of claim 35, wherein said ratio is greater than 10 to 1.

37. The process of claim 35, wherein said ratio is greater than 12 to 1 and less than 15 to 1.

38. The process of claim 31, wherein said reacting is conducted at a temperature above 40°C.

39. The process of claim 31, wherein said reacting is conducted at a temperature within the range of 45°C to 55°C.

40. The process of claim 31, wherein said weight fraction of said nitric acid is within a range of 0.42 to 0.50, and said weight fraction of said sulfuric acid is within a range of 0.58 to 0.50.
